(19) Europäisches Patentamt
European Patent Office
Office européen des brevets

(11) Veröffentlichungsnummer: **0 232 712 B1**

# (12) EUROPÄISCHE PATENTSCHRIFT

(45) Veröffentlichungstag der Patentschrift: **26.09.90**

(51) Int. Cl.[5]: **C07C 253/20**, C07C 253/22, C07C 255/06

(21) Anmeldenummer: **87100166.5**

(22) Anmeldetag: **08.01.87**

## (54) Verfahren zur Herstellung von Nitrilen.

(30) Priorität: **14.01.86 DE 3600811**

(43) Veröffentlichungstag der Anmeldung: **19.08.87 Patentblatt 87/34**

(45) Bekanntmachung des Hinweises auf die Patenterteilung: **26.09.90 Patentblatt 90/39**

(84) Benannte Vertragsstaaten: **BE CH DE FR GB LI NL**

(56) Entgegenhaltungen:
**EP-A- 0 196 554**

**CHEMICAL ABSTRACTS, Band 101, Nr. 3, 16. Juli 1984, Seite 529, Nr. 22606h, Columbus, Ohio, US; A.V.RAO et al.: "Synthesis of nitriles from carboxamides with zeolites"**
**CHEMICAL ABSTRACTS, Band 84, Nr. 1, 5. Januar 1976, Seite 379, Nr. 4433a, Columbus, Ohio, US; E.COSTA NOVELLA et al.: "Nitrilation of pelargonic acid"**

(73) Patentinhaber: **BASF Aktiengesellschaft, Carl-Bosch-Strasse 38, D-6700 Ludwigshafen(DE)**

(72) Erfinder: **Hoelderich, Wolfgang, Dr., Mannheimer Strasse 18 c, D-6710 Frankenthal(DE)**
Erfinder: **Fischer, Rolf, Dr., Bergstrasse 98, D-6900 Heidelberg(DE)**
Erfinder: **Vagt, Uwe, Dr., Paul-Neumann-Strasse 44, D-6720 Speyer(DE)**

EP 0 232 712 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von γ,δ-ungesättigten Nitrilen durch katalytische Dehydratisierung von γ,δ-ungesättigten Carbonsäureamiden.

Es ist z.B. aus Houben-Weyl, Methoden der Organischen Chemie, 4. Aufl., Bd VIII, Seiten 334 u. 337–338 (1952) bekannt, Nitrile durch thermische Wasserabspaltung aus Säureamiden oder Carbonsäuren bzw. deren Derivaten und Ammoniak in Gegenwart geeigneter Katalysatoren herzustellen. Als Katalysatoren werden z.B. Aluminiumoxid, Siliciumdioxid, Manganoxid, Thoriumoxid oder Graphitkatalysatoren verwendet. Zur Erzielung hoher Umsätze und Ausbeuten sind allerdings Temperaturen von 400 bis 500°C erforderlich. Die bekannten Verfahren haben ferner den Nachteil, daß die Katalysatoren schnell desaktivieren und ihre Selektivität einbüßen.

Die Dehydratisierung einfach strukturierter, thermisch sehr stabiler Carbonsäureamide wie Benzamid oder Phenylacetamid an ZSM-5-Zeolithen bei 400°C is z.B in Chemistry and Industry, S. 270 (1984) beschrieben.

Der Erfindung lag daher die Aufgabe zugrunde, ein einfaches und wirtschaftliches Verfahren zu finden, nach dem γ,δ-ungesättigte Carbonsäureamide durch katalytische Wasserabspaltung in die entsprechenden Nitrile überführt werden können, ohne daß eine Isomerisierung der Doppelbindung stattfindet. Das Verfahren sollte sich durch hohe Selektivität und hohen Umsatz bei langen Katalysatorstandzeiten auszeichnen. Vorteilhaft sollten die Amide in situ aus Carbonsäuren bzw. deren Derivaten und Ammoniak hergestellt werden können.

Es wurde nun ein Verfahren zur Herstellung von Nitrilen durch katalytische Dehydratisierung von Carbonsäureamiden gefunden, das dadurch gekennzeichnet ist, daß man γ,δ-ungesättigte Carbonsäureamide in Gegenwart von Zeolithen und 0,01 bis 50 Mol Ammoniak je Mol Säureamid dehydratisiert.

Das erfindungsgemäße Verfahren läßt sich z.B. für die Umsetzung von 40-Pentensäureamid zu 4-Pentennitril durch folgende Reaktionsgleichung wiedergegeben:

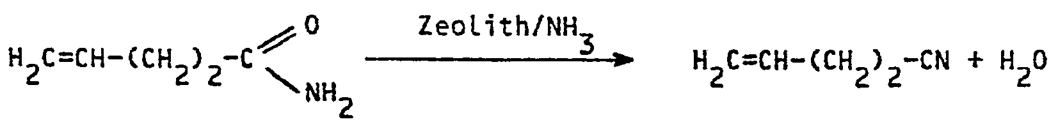


Durch die Maßnahme, die Dehydratisierung an Zeolithen in Gegenwart von Ammoniak durchzuführen, wird eine bemerkenswerte Selektivitätserhöhung (s. Vergleichsbeispiel 1) und eine Steigerung der Katalysatorstandzeit erreicht. Zum anderen ist es dadurch möglich, die Carbonsäureamide in situ aus deren Vorstufen wie z.B. Carbonsäuren, Säurehalogeniden oder Estern und überschüssigem Ammoniak herzustellen. Das neue Verfahren hat weiterhin den Vorteil, daß sich die Katalysatoren leicht regenerieren lassen und auch nach mehrmaligem Regenerieren eine hohe Katalysatoraktivität und Selektivität aufweisen.

Eine Isomerisierung der inden Ausgangsstoffen vorliegenden Doppelbindung findet überraschenderweise nicht oder nur in geringem Ausmaß statt.

Beispielsweise können folgende ungesättigte Ausgangsstoffe verwendet werden:

4-Pentensäureamid, 4-Pentensäuremethylester, 4-Pentensäure, 2-Methyl-4-pentensäure, 2-Methyl-4-pentensäureammid, 3-Methyl-4-pentensäureethylester, 3,3-Dimethyl-4-pentensäurepropylester, 3,3-Dimethyl-4-pentensäure oder 3,3-Dimethyl-4-pentensäure-methylestersäure.

Als Katalysatoren für die erfindungsgemäße Herstellung von Alkennitrilen werden acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern aufweisen, die durch gemeinsame Sauerstoffatome verbunden sind. Das Verhältnis der Si- und Al-Atome zu Sauerstoff beträgt 1:2 [siehe Ullmanns Encyclopädie der techn. Chemie, 4. Auflage, Band 24, Seite 575 (1983)]. Die Elektrovalenz der Aluminum enthaltenden Tetraeder ist durch Einschluß von Kationen in den Kristall, z.B. eines Alkali- oder Wasserstoffions ausgeglichen, ein Kationenaustausch ist möglich.

In den Zeolithen können anstelle von Aluminium auch andere Elemente wie B, Ga, Fe, Cr, V, As, Sb in das Gitter eingebaut werden oder das Silicium kann durch ein vierwertiges Element wie Ge ersetzt werden.

Als Katalysatoren kommen Zeolithe aus der Faujasit-Gruppe, z.B. der Zeolith Y oder aus der Mordenit-Gruppe oder engporige Zeolithe z.B. vom Erionit- bzw. Chabasit-Typ in Betracht. Besonders vorteilhaft für das erfindungsgemäße Verfahren sind Zeolithe vom Pentasil-Typ. Diese Zeolithe können unterschiedliche chemische Zusammensetzungen aufweisen, z.B. handelt es sich hierbei um Alumino-, Boro-, Gallium- und Eisengermanatzeolithe oder deren Gemische sowie um Gallium-, Chrom-, Eisen-, Antimon-, Bismut und insbesondere um Alumino-, Boro- und Eisensilikatzeolithe oder deren Gemische.

Der Aluminiumsilikatzeolith wird z.B. aus einer Aluminiumverbindung, vorzugsweise $Al(OH)_3$ oder $Al_2(SO_4)_2$ und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit oder ohne Alkali- oder Erdalkalizusatz bei 100 bis 220°C unter autogenem Druck hergestellt. Auch gehören

hierzu die isotaktischen Zeolithe nach DE-OS 30 06 471. Die erhaltenen Aluminiumsilikatzeolithe können je nach Wahl der Einsatzstoffmengen ein $SiO_2/Al_2O_3$-Verhältnis von 10 bis 40.000 enthalten. Auch lassen sich derartige Aluminosolikatzeolithe in etherischem Medium wie Diethylenglykoldimethylether, in alkoholischem Medium wie Methanol bzw. 1,4-Butandiol oder in Wasser synthetisieren.

Der Borosilikatzeolith kann z.B. bei 90 bis 200°C unter autogenem Druck synthetisiert werden, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit oder ohne Alkali- oder Erdalkalizusatz zur Reaktion bringt. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Die so hergestellten Alumino-, Boro- und Eisensilikatzeolithe können nach ihrer Isolierung, Trocknung bei 100 bis 160°C, vorzugsweise 100°C und Calcinierung bei 450 bis 550°C, vorzugsweise 500°C, mit einem Bindemittel, z.B. im Verhältnis 90:10 bis 40:60 Gew.%, zu Strängen oder Tabletten verformt werden. Als Bindemittel eignen sich z.B. Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und $Al_2O_3$, hochdisperses $TiO_2$ sowie Ton. Nach der Verformung werden die Extrudate oder Preßlinge getrocknet und calciniert.

Man erhält auch vorteilhafte Katalysatoren, wenn der isolierte Alumino- bzw. Boro- bzw. Eisensilikatzeolith direkt nach der Trocknung verformt wird und erstmals nach der Verformung einer Calcinierung unterworfen wird. Die hergestellten Alumino-, Boro- und Eisensilikatzeolithe können aber auch in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abtrennen der Koksablagerung mit Luft oder mit einem Luft/$N_2$-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Acidität des Katalysators für ein Selektivitätsoptimum des gewünschten Reaktionsproduktes einzustellen.

Um eine möglichst große Selektivität, hohen Umsatz sowie lange Standzeiten zu erreichen, ist es mitunter vorteilhaft, die Zeolithe zu modifizieren. Eine geeignete Modifizierung der Katalysatoren besteht z.B. darin, daß man den unverformten Zeolithen mit Metallsalzen durch einen Ionenaustausch oder durch Imprägnierung dotiert.

Für eine Dotierung der Zeolithe kommen z.B. Alkalimetalle wie Li, Na, Cs,Übergangsmetalle wie Mo, Fe, Zn, Cu und vor allem W, Edelmetalle wie Pd oder Pt und seltene Erdmetalle wie La und Ce in Betracht.

Zweckmäßigerweise führt man die Dotierung so durch, daß man z.B. den verformten Pentasilzeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cernitrat in Wasser löst. Mit dieser Lösung wird der verformte oder unverformte Zeolith eine gewisse Zeit, ca. 30 Minuten, getränkt. Die eventuell überstehende Lösung wird am Rotationsverdampfer vom Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniakali-

sche $Pd(NO_3)_2$-Lösung bei Temperaturen von 3O bis 8O°C im Kreislauf 15 bis 2O h leitet. Danach wird mit Wasser ausgewaschen, getrocknet und bei ca. 55O°C calciniert.

Bei manchen metalldotierten Zeolithen, z.B. Pd- oder Pt-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C 16 Stunden getrocknet und bei 500°C 20 Stunden caciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3-bis 25 gew.%igen, insbesondere 12- bis 20 gew.%igen wäßrigen Salzsäure. Anschließend wird der so behandelte Zeolith mit Wasser gewaschen, getrocknet und bei 400 bis 500°C. calciniert.

Nach einer anderen Arbeitsweise lassen sich Zeolithe durch Aufbringung von Phosphorverbindungen, wie Trimethoxyphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form wit wäßriger $NaH_2PO_4$-Lösung getränkt, bei 110°C getrocknet und bei 500°C calciniert.

Im allgemeinen können die Kataylysatoren wahlweise als Stränge von 2 bis 4 mm Länge, als Tabletten mit 3 bis 5 mm Durchmesser oder als Wirbelgut mit einer Teilchengröße von 0,05 bis 0,5 mm verwendet. Das Wirbelgut läßt sich z.B. durch Zerkleinern und Aussieben von Strängen oder durch Sprühtrocknung herstellen.

Die Umsetzung an Zeolithen in Gegenwart von Ammoniak durchgeführt. Dabei können Ammoniakmengen von 0,01 bis 50, insbesondere 1 bis 30, vorzugsweise 3 bis 20 Mol Ammoniak je Mol Amid verwendet werden. Werden die Carbonsäureamide in situ z.B. aus Carbonsäuren oder deren Derivaten und Ammoniak erzeugt, so haben sich Ammoniakmengen von 1 bis 50, insbesondere 5 bis 30 mol je Mol Ausgangsstoff bewährt. Höhere Ammoniakmengen sind möglich, jedoch nicht wirtschaftlich.

Die Umsetzung kann in der Gasphase oder in der flüssigen Phase z.B unter Mitverwendung von Verdünnungsmitteln durchgeführt werden. Vorzugsweise wird in der Gasphase umgesetzt, sofern gut verdampfbare Ausgangsstoffe verwendet werden.

Die Reaktionstemperatur liegt im allgemeinen bei 50 bis 450°C. Falls in der Flüssigphase dehydrtisiert wird, beträgt die Temperatur vorteilhaft 50 bis 300, insbesondere 100 bis 200°C. In der Gasphase werden in der Regel höhere Temperaturen von 150 bis 450, insbesondere 200 bis 350°C gewählt. Die Katalysatorbelastung (WHSV) liegt im allgemeinen bei 0,01 bis 4O, vorzugsweise O,O5 bis 15 g Carbonsäurederivat je Gramm Katalysator und Stunde.

Das Verfahren kann kontinuierlich oder diskontinuierlich drucklos oder bei Überdruck nach den dafür üblichen Techniken durchgeführt werden. Auch ist die Umsetzung in der Flüssigphase in Gegenwart von unter den Reaktionsbedindungen inerten Lösungsmitteln, z.B. Ethern wie Diethylether, Tetrahydrofuran oder Dioxan, oder in der Gasphase in Gegenwart von Inertgasen, z.B. Stickstoff, möglich.

Die Aufarbeitung und Isolierung der Reaktionsprodukte erfolgt in an sich bekannter Weise, z.B. destillativ, so daß sich Ausführungen hierzu erübrigen. Nicht umgesetzte Carbonsäurederivate können abgetrennt und erneut für die erfindungsgemäße Umsetzung verwendet werden.

Die nach dem erfindungsgemäßen Verfahren erhältlichen Alkennitrile der Formel I stellen wertvolle Zwischenprodukte für die Herstellung von Faservorprodukten, Pflanzenschutzmitteln und Pharmazeutika dar. Ausgehend von 4-Pentennitril läßt sich z.B. durch Addition von Blausäure Adipodinitril einfacher und mit höherer Selektivität herstellen als z.B. ausgehend von 3-Pentennitril. Durch Carbonylierung von 4-Pentennitril sind weiterhin 5-Cyanvaleriansäureester, Vorprodukte für die Herstellung von Caprolactam, zugänglich (DE-OS 2 541 64O).

## Beispiele

### Herstellung der Katalysatoren

### Katalysator A

Der Borzeolith des Pentasiltyps wurde in einer hydrothermalen Synthese aus 64O g hochdispersem $SiO_2$, 122 g $H_3BO_3$, 8OOO g einer wäßrigen 1,6-Hexandiamin-Lösung (Mischung 5O:5O Gew.%) bei 17O°C unter autogenem Druck in einem Rührautoklaven hergestellt. Nach Abfiltrieren und Auswaschen wurde das kristalline Reaktionsprodukt 24 h bei 1OO°C getrocknet und 24 h bei 5OO°C calciniert. Dieser Borosilikatzeolith enthielt 94,2 Gew.% $SiO_2$ und 2,3 Gew.% $B_2O_3$.

Mit diesem Material wurden durch Verformen mit Boehmit im Gewichtsverhältnis 6O:4O 2-mm-Stränge hergestellt, die 16 h bei 11O°C getrocknet und 24 h bei 5OO°C calciniert wurden.

Katalysator B

Kommerziell erhältlicher NaY-Zeolith wurde in einer Kolonne vorgelegt und mit 20 %iger Ammoniumchlorid-Lösung im Gewichtsverhältnis 1:15 bei 80°C 2 h behandelt. Dieser Vorgang wurde solange wiederholt, bis der Na-Wert des bei 110°C getrockneten und 5 h bei 500°C calcinierten Y-Zeolithen auf 0,13 Gew.% gesunken war.

Beispiele 1 und 2

Herstellung von 4-Pentennitril

Die Umsetzung wurde in der Gasphase unter isothermen Bedingungen in einem Rohrreaktor jeweils 6 Stunden lang durchgeführt. Hierzu wurden pro Stunde 5 ml einer 10 gew.%igen 4-Pentensäureamidlösung in Tetrahydrofuran verdampft und mit einem Gasstrom (2 l/h Stickstoff und 10 l/h Ammoniak) bei 250°C über jeweils 10 g Katalysator geführt. Die Versuchsergebnisse sind in Tabelle 1 zusammengestellt. Im Vergleichsbeispiel 1a wurde die Umsetzung entsprechend Beispiel 1, jedoch ohne Zugabe von Ammoniak bei einem Gasstrom von 12 l/h Stickstoff durchgeführt. Die Reaktionsprodukte wurden durch übliche Methoden abgetrennt und charakterisiert. Die quantitative Bestimmung der Reaktionsprodukte und Ausgangsstoffe erfolgte gaschromatographisch.

Tabelle 1

| Beispiel | 1 | 1a | 2 |
|---|---|---|---|
| Katalysator | A | A | B |
| Produktzusammensetzung (mol%) | | | |
| 4-Pentennitril | 99 | 58 | 81 |
| 2-Pentennitril | Spuren | 9 | Spuren |
| Pentensäureamide | " | 6 | 9 |
| Pentensäure | " | 3 | Spuren |
| 5-Methylbutyrolactron | " | 20 | " |

Die oben beschriebene Umsetzung verlief in etwas geringerer Ausbeute, wenn ein Aluminiumsilikatzeolith des Pentasiltyps (90 Gew.% $SiO_2$, 3,5 Gew.% $Al_2O_3$) oder ein mit Cer oder Lanthan dotierter Katalysator A verwendet wurde.

Beispiel 3

In einem Standzeitversuch wurden unter den Bedingungen von Beispiel 1 pro Stunde 5 ml einer 10 gew.%igen 4-Pentensäureamidlösung in Tetrahydrofuran verdampft und mit einem Gasstrom (2 l/h Stickstoff und 10 l/h Ammoniak bei 250°C über 10 g Katalysator A geführt. Innerhalb von 152 h wurde keine Abnahme der 4-Pentennitril-Ausbeute (93 % Selektivität bei 99 % Umsatz) beobachtet.

Beispiel 4

Ein 100 cm langes Quarzrohr (Innendurchmesser 20 mm) mit zwei getrennten elektrischen Heizungen wurde im unteren Teil mit 180 g Katalysator A (Temperatur 251 bis 258°C, Reaktionszone) und im oberen Teil mit Glasringen (Temperatur 230 bis 240°C, Verdampferzone) gefüllt. 1200 ml einer 10 gew.%igen 4-Pentensäureamidlösung in Tetrahydrofuran wurden mit 180 ml/h von oben auf den Verdampfer gepumpt und mit einem Gasstrom (15 l/h Stickstoff und 30 l/h Ammoniak) durch das Quarzrohr geleitet. Nach üblicher Aufarbeitung und fraktionierender Destillation wurden 77,7 g (Ausbeute 90 % d. Th.) 4-Pentennitril als farblose Flüssigkeit vom Sdp. 142 bis 143°C erhalten.

**Patentansprüche**

1. Verfahren zur Herstellung von Nitrilen durch katalytische Dehydratisierung von Carbonsäureamiden, dadurch gekennzeichnet, daß man $\gamma,\delta$-ungesättigte Carbonsäureamide in Gegenwart von Zeolithen und 0,01 bis 50 mol Ammoniak je Mol Säureamid dehydratisiert.

2. Verfahren nach Anspruch 1, dadurch gekennzeichnet, daß man als Katalysatoren Zeolithe des Pentasiltyps verwendet.

3. Verfahren nach den Ansprüchen 1 und 2, dadurch gekennzeichnet, daß man als Katalysatoren bor-, Eisen- oder Aluminiumsilikatzeolithe verwendet.

4. Verfahren nach Anspruch 1, dadurch gekennzeichnet, daß man als Katalysatoren Aluminiumsilikatzeolithe des Y-Typs verwendet.

5. Verfahren nach den Ansprüchen 1 bis 4, dadurch gekennzeichnet, daß man als Katalysatoren mit seltenen Erdmetallen oder mit Übergangsmetallen dotierte Zeolithe verwendet.

**Claims**

1. A process for preparing a nitrile by catalytic dehydration of a carboxamide, which comprises dehydrating a γ,δ-unsaturated carboxamide in the presence of a zeolite and from 0.01 to 50 mol of ammonia per mole of carboxamide.

2. A process as claimed in claim 1, wherein the catalyst used is a zeolite of the pentasil type.

3. A process as claimed ineither of claims 1 and 2, whereinthe catalyst used is a boron, iron or aluminium silicate zeolite.

4. A process as claimed in claim 1, wherein the catalyst used is an aluminium silicate zeolite of the Y-type.

5. A process as claimed in any of claims 1 to 4, wherein the catalyst used is a zeolite doped with a rare earth metal or with a transition metal.

**Revendications**

1. Procédé d'obtention de nitriles par déshydratation catalytique d'amides d'acides carboxyliques, caractérisé en ce qu'on déshydrate des amides d'acides carboxyliques à insaturation γ,δ en présence de zéolites et de 0,01 à 50 moles d'ammoniac par mole d'amide.

2. Procédé selon la revendication 1, caractérisé en ce qu'on utilise comme catalyseurs des zéolites du type Pentasil.

3. Procédé selon les revendications 1 et 2, caractérisé en ce qu'on utilise comme catalyseurs des zéolites de silicates de bore, de fer ou d'aluminium.

4. Procédé selon la revendication 1, caractérisé en ce qu'on utilise comme catalyseurs des zéolites de silicate d'aluminium du type Y.

5. Procédé selon les revendications 1 à 4, caractérisé en ce qu'on utilise comme catalyseurs des zéolites dopés avec des métaux de terres rares ou des métaux de transition.